# EUROPEAN PATENT SPECIFICATION

(11) **EP 3 246 309 B1**
(45) Date of publication and mention of the grant of the patent: **01.01.2020**
(21) Application number: 16737383.6
(22) Date of filing: 13.01.2016
(51) Int. Cl.: C07C 213/10, C07C 213/02, C07C 219/32, C07D 307/58

(54) **METHOD FOR TREATING TIN COMPOUND IN REACTION MIXTURE**
VERFAHREN ZUR BEHANDLUNG EINER ZINNVERBINDUNG IN EINEM REAKTIONSGEMISCH
PROCÉDÉ DE TRAITEMENT D'UN COMPOSÉ DE L'ÉTAIN DANS UN MÉLANGE RÉACTIONNEL

(30) Priority: 13.01.2015 JP 2015004367
(43) Date of publication of application: 22.11.2017
(73) Proprietor: Nissan Chemical Corporation, Tokyo (JP)
(72) Inventor: NAGAO, Masato, Funabashi-shi Chiba 274-0052 (JP); GOTO, Yuichi, Toyama-shi Toyama 939-2753 (JP)
(74) Representative: Blodig, Wolfgang
(86) International application number: PCT/JP2016/050854
(87) International publication number: WO 2016/114312

(56) References cited:
- WO-A1-98/52990
- WO-A1-2008/096145
- WO-A1-2012/002511
- WO-A1-2012/002513
- WO-A1-2014/208609
- JP-A- H02 275 857
- JP-A- 2002 506 466
- JP-A- 2010 518 060

## Description

### TECHNICAL FIELD

The present invention relates to a method for simply and efficiently treating a tin compound in a reaction mixture.

### BACKGROUND ART

As methods for removing residues of a Sn compound used as a catalyst at the time of reducing an aromatic nitro compound as a starting material by means of SnCl₂ (stannous chloride) to produce an aromatic amine compound, the following methods have been known.

Patent Document 1 and Non-Patent Document 1 disclose a method of adding a sodium hydrogen carbonate solution to a reaction mixture after a reaction for neutralization and filtering precipitates derived from Sn.

Patent Document 2 discloses a method of distilling away a solvent after a reaction, adding a sodium hydroxide aqueous solution to residues, controlling pH to about from 12 to 14 and then extracting a product with ethyl acetate.

Patent Document 3 discloses a method of adding sodium hydroxide to a reaction liquid so as to be strong basic and extracting a product with diethyl ether.

Patent Document 4 discloses a method of adding a potassium hydroxide aqueous solution to a reaction liquid which is an ethyl acetate solution, extracting a product with ethyl acetate and then purifying the-product by silica gel chromatography.

Further, a method has been known that SnCl₂ is used as a reaction agent in a reaction to react acetal and α-bromoester.

As the purification method after a reaction, Patent Document 5 discloses a method of extracting a desired product from a solution after a reaction by using an organic solvent and carrying out recrystallization. However, as the result of studies by the present inventors, it has been found that in the above method, a large amount of Sn remains in crystals.

### PRIOR ART DOCUMENTS

### PATENT DOCUMENTS

Patent Document 1: WO2013/099804
Patent Document 2: JP-A-2012-526061
Patent Document 3: JP-A-2010-530405
Patent Document 4: JP-A-2008-526910
Patent Document 5: WO2012/002513

### NON-PATENT DOCUMENT

Non-Patent Document 1: J. Org. Chem. year 2005, Vol. 70, pages 63 to 78 WO 98/52990 discloses fluorinated amine products.
WO2008/096145 A1 discloses a process for preparing anagrelide.

### DISCLOSURE OF INVENTION

### TECHNICAL PROBLEM

It is an object of the present invention to provide a novel method for simply and efficiently removing a Sn compound in a reaction liquid in a reaction by means of SnCl₂ (stannous chloride).

### SOLUTION TO PROBLEM

The present inventors have extensively studied in order to accomplish the above object, and as a result, they have found the followings.
1. A method for producing a compound B, which comprises reacting a compound A as a starting material by means of stannous chloride in accordance with the following reaction formula (1) and which includes a purification step of bringing the above reaction liquid into contact with a mixed liquid of a compound having a benzene ring and a potassium hydroxide aqueous solution to carry out liquid separation and obtaining the compound B from the obtained organic layer: 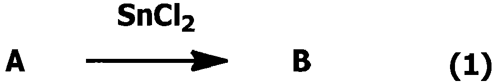 where the compound A may be a single compound or a mixture of plural compounds.
2. The method according to the above 1, wherein the method of bringing the reaction liquid into contact with the mixed liquid of a compound having a benzene ring and a potassium hydroxide aqueous solution is a method of adding the reaction liquid into the mixed liquid of a compound having a benzene ring and a potassium hydroxide aqueous solution.
3. The method according to the above 1, wherein the reaction represented by the reaction formula (1) is a reduction reaction or a lactone ring-forming reaction by means of stannous chloride.
4. The method according to the above 3, wherein the compound A is an aromatic dinitro compound represented by the formula 1, and the compound B is an aromatic diamine compound represented by the formula 2: 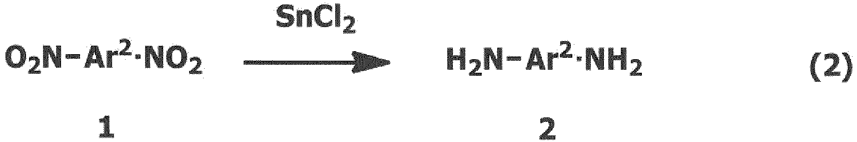 were Ar² is an aromatic group.
5. The method according to the above 4, wherein the compound represented by the formula 1 is an aromatic dinitro compound represented by the formula 3: 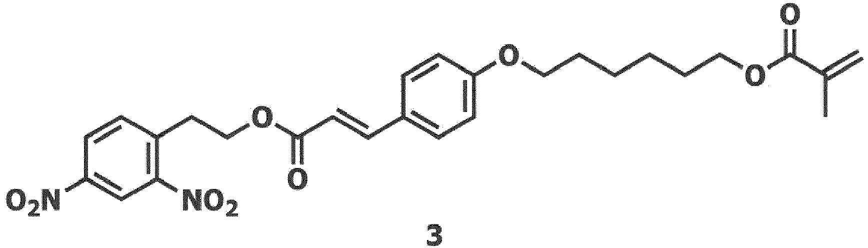
6. The method according to the above 3, wherein the compound A is a mixture of an acetal compound represented by the formula 4 and an ester compound represented by the formula 5, and the compound B is an α-methylene-γ-butyrolactone compound: 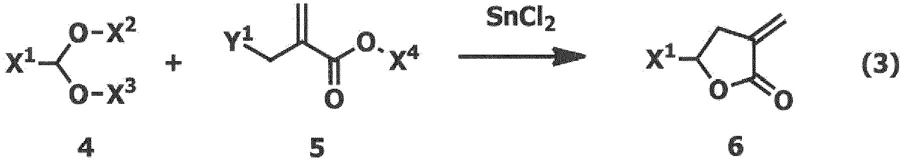 where X¹ is an organic group, X² and X³ are each independently a C₁₋₄ alkyl group, or X² and X³ may together form CH₂CH₂ or CH₂CH₂CH₂, X⁴ is a C₁₋₄ alkyl group, and Y¹ is Cl or Br.
7. The method according to the above 6, wherein the compound represented by the formula 4 is an acetal compound represented by the formula 7: 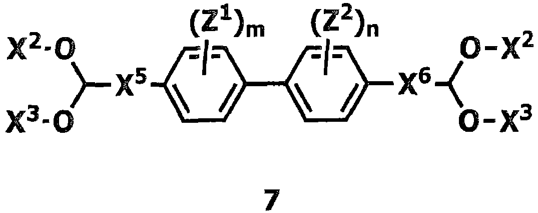 where X² and X³ are each independently a C₁₋₄ alkyl group, or X² and X³ may together form CH₂CH₂ or CH₂CH₂CH₂, X⁵ and X⁶ are each independently a C₁₋₁₀ alkylene group bonding to a benzene ring via an etheric bond or a single bond, Z¹ and Z² are each independently a substituent selected from a halogen atom, an alkyl group, a haloalkyl group, an alkoxy group and a haloalkoxy group, m and n are each independently an integer of from 0 to 4, and in a case where at least one of m and n is 2 or higher, two or more Z¹ and/or Z² may be the same or different from each other.
8. The method according to any one of the above 1 to 7, wherein the concentration of the potassium hydroxide aqueous solution is from 1 to 50 mass%.
9. The method according to any one of the above 1 to 8, wherein the amount of potassium hydroxide is at least 3 times by mol per 1 mol of tin atoms.
10. The method according to any one of the above 1 to 9, wherein the temperature to carry out the liquid separation is from 10 to 80°C.
11. The method according to any one of the above 1 to 10, wherein the ratio in amount to be used of the potassium hydroxide aqueous solution to the compound having a benzene ring is from 50:1 to 1:50 by mass ratio.
12. The method according to any one of the above 1 to 11, wherein the compound having a benzene ring has a boiling point of from 80 to 170°C.
13. The method according to any one of the above 1 to 12, wherein the compound having a benzene ring is toluene.
14. The method according to any one of the above 1 to 13, wherein a reaction solvent is further used.
15. The method according to the above 14, wherein the reaction solvent is tetrahydrofuran.
16. The method according to the above 14, wherein the reaction solvent is water.

### ADVANTAGEOUS EFFECTS OF INVENTION

According to the present invention, in a reaction by means of SnCl₂ (stannous chloride), a novel method for simply and efficiently removing a tin compound in a reaction liquid is provided, and a highly pure desired product can be obtained.

### DESCRIPTION OF EMBODIMENTS

The present invention is in accordance with the following reaction formula (1). That is, in a method for producing a compound B, which comprises reacting a compound A as a starting material by means of stannous chloride, a purification step of bringing the above reaction liquid into contact with a mixed liquid of a compound having a benzene ring and a potassium hydroxide aqueous solution to carry out liquid separation, is included:

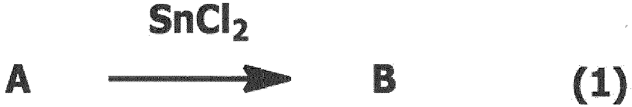

Here, the compound A may be a single compound or a mixture of plural compounds.

In a case where the compound A is an aromatic nitro compound or an aromatic dinitro compound, in accordance with the following reaction formula (4) or (2), an aromatic nitro compound represented by the formula 8 or an aromatic dinitro compound represented by the formula 1 (Ar and Ar² are aromatic groups) is reduced in the presence of stannous chloride, and an aromatic amine compound represented by the formula 9 or an aromatic diamine compound represented by the formula 2 is formed:

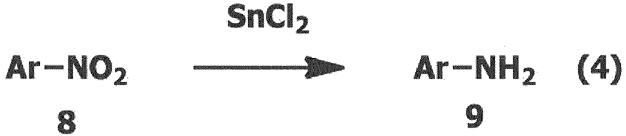

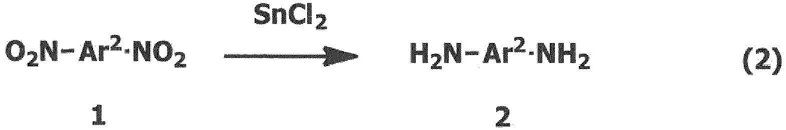

The above dinitro compound may have two NO₂ groups at ortho, meta or para, and preferably at meta.

In the present invention, as examples of the aromatic nitro compound or the aromatic dinitro compound represented by the formula 1 or the formula 8, the following formulae 1-1 to 1-53 may be mentioned.

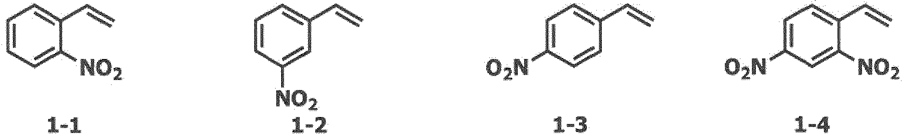

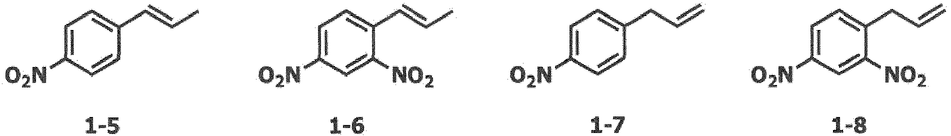

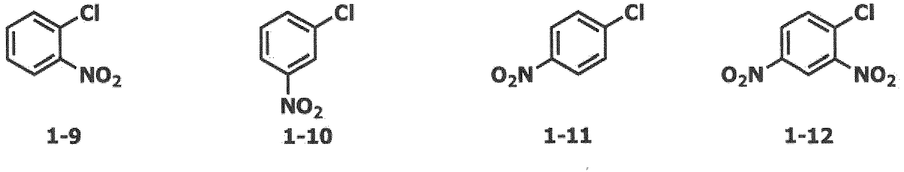

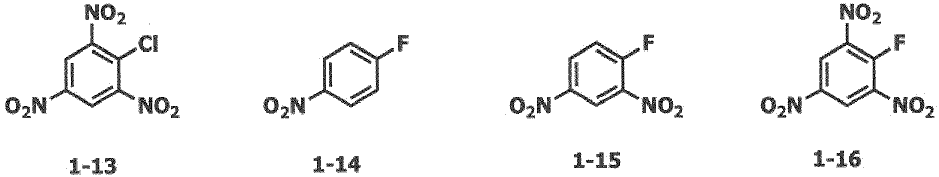

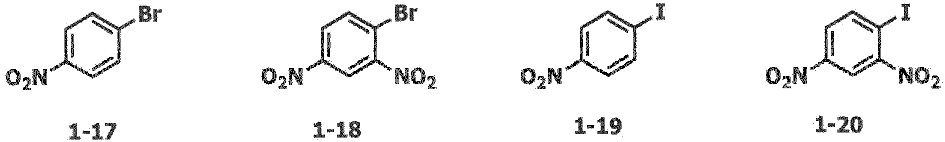

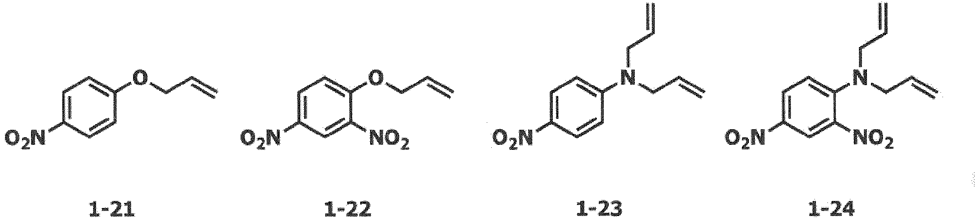

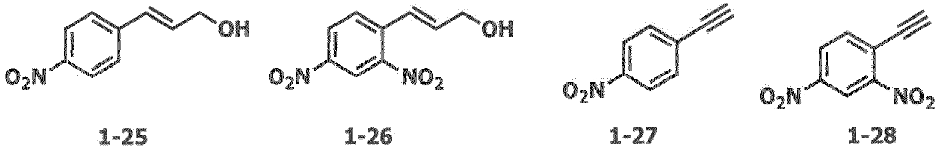

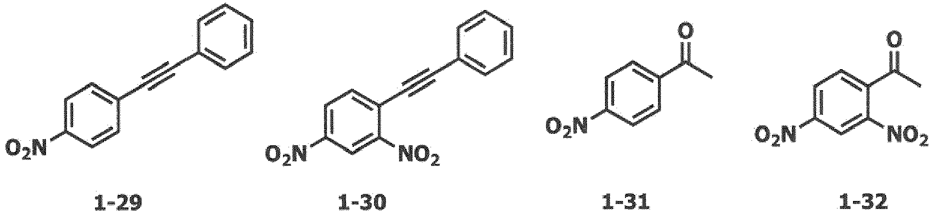

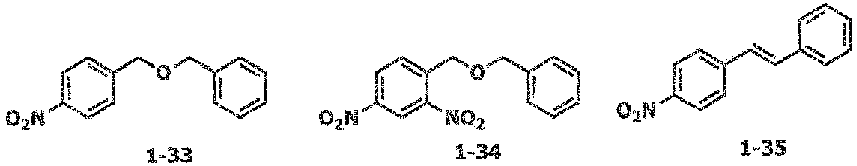

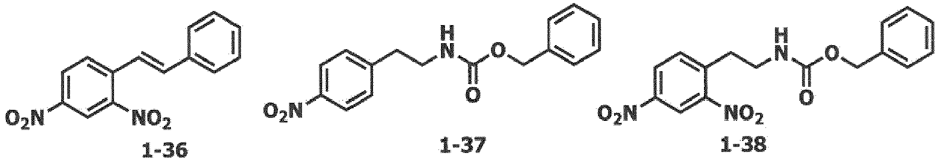

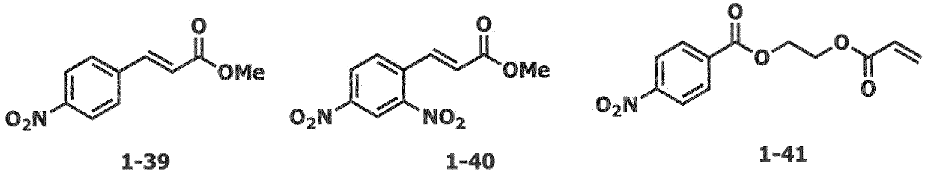

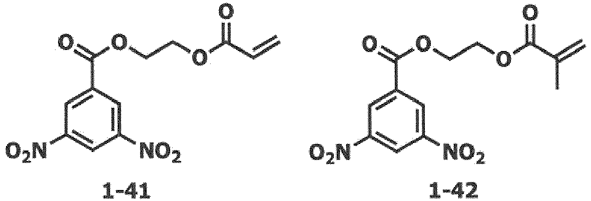

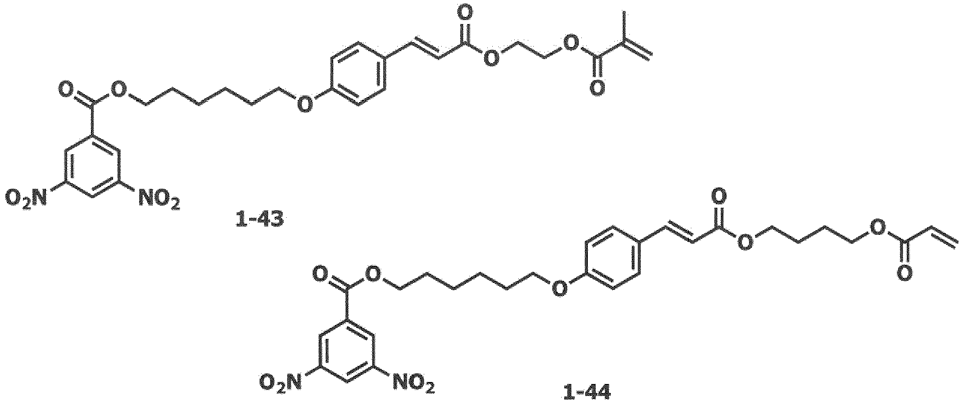

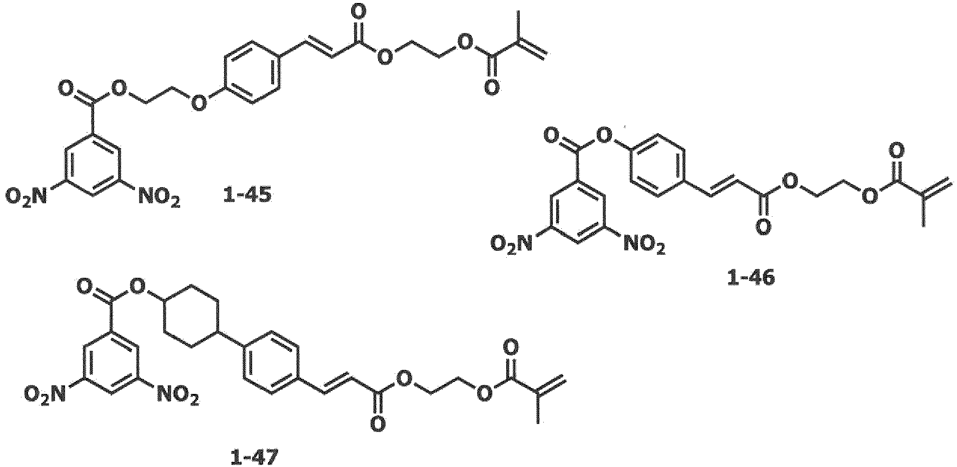

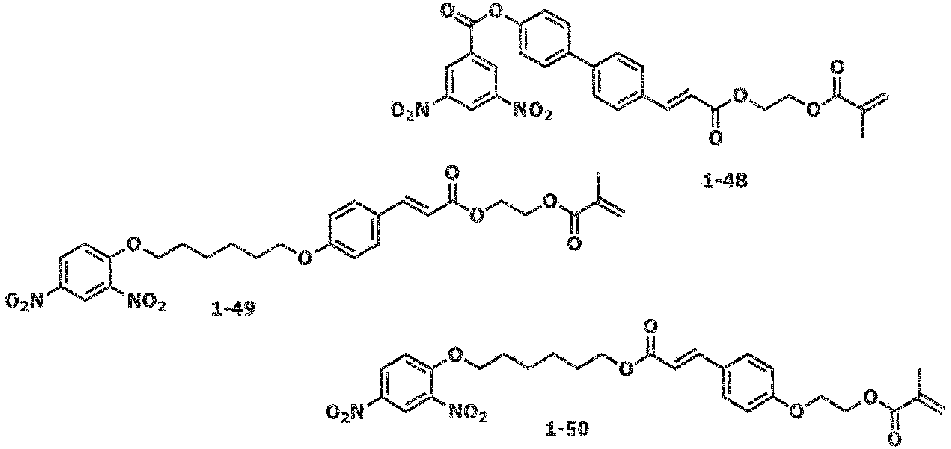

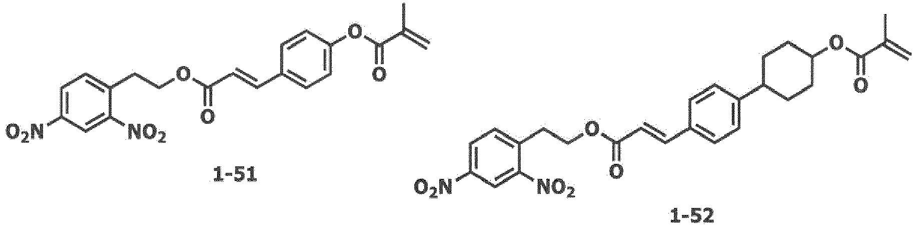

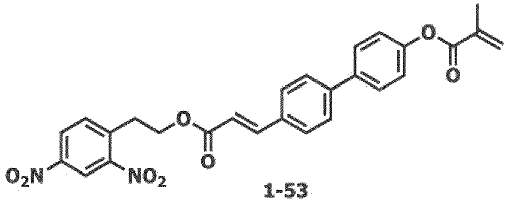

Further, in the present invention, as other examples of the aromatic nitro compound or the aromatic dinitro compound represented by the formula 1or the formula 8, the dinitro compounds mentioned in WO2007/071091, p.81, the compounds (where diamino shall be read as dinitro) mentioned in WO2007/071091, p.82 to p.88, the dinitro compound mentioned in WO2007/071091, p.88, the compounds (where diamino shall be read as dinitro) mentioned in WO2007/071091, p.89 to p.92, the dinitro compound mentioned in WO2007/071091, p.93, the compounds (where diamino shall be read as dinitro) mentioned in WO2007/071091, p.94 to p.99, the dinitro compounds mentioned in WO2007/071091, p.101, the compounds (where amino shall be read as nitro) mentioned in WO2007/071091, p.102 to p.104, the dinitro compounds mentioned in WO2007/071091, p.104, the dinitro compounds mentioned in WO2007/071091, p.107, the compounds (where diamino shall be read as dinitro) mentioned in WO2007/071091, p.109 to p.110, the dinitro compounds mentioned in WO2007/071091, p.111, the compounds (where diamino shall be read as dinitro) mentioned in WO2007/071091, p.112 to p.115, the dinitro compounds mentioned in WO2008/145225, p.87 and p.88, the compounds (where diamino shall be read as dinitro) mentioned in WO2008/145225, p.89 to p.93, the dinitro compounds mentioned in WO2008/145225, p.93, the compounds (where diamino shall be read as dinitro) mentioned in WO2008/145225, p.95 to p.106, the dinitro compounds mentioned in WO2008/145225, p.106, the compounds (where diamino shall be read as dinitro) mentioned in WO2008/145225, p.108 to p.135, the dinitro compounds mentioned in WO2008/145225, p.137, the compounds (where amino shall be read as nitro) mentioned in WO2008/145225, p.139 to p.141, the dinitro compounds mentioned in WO2008/145225, p.143, the dinitro compounds mentioned in WO2008/145225, p.145, the compounds (where amino shall be read as nitro) mentioned in WO2008/145225, p.146 to p.153 and the dinitro compounds mentioned in WO20131099804, p.51, p.55, p.58, p.60, p.63, p.65, p.68, p.71 and p.74 may be mentioned.

Among them, as the compound represented by the formula 1, the dinitro compound represented by the following formula 3 may be preferably mentioned:

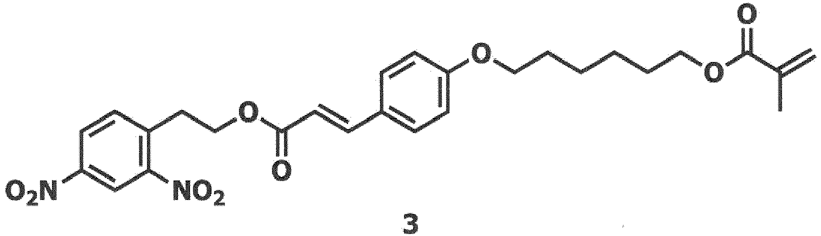

The above reduction reaction may be carried out by using or not using a reaction solvent, however, the reduction reaction is preferably carried out by using a reaction solvent. The reaction solvent is not particularly restricted, so long as it is inert to the reaction.

For example, a hydrocarbon such as hexane, cyclohexane, benzene or toluene; a halogenated hydrocarbon such as carbon tetrachloride, chloroform or 1,2-dichloroethane; an alcohol such as methanol, ethanol or isopropyl alcohol; an ether such as diethyl ether, diisopropyl ether, 1,4-dioxane or tetrahydrofuran; a ketone such as acetone, methyl ethyl ketone or methyl isobutyl ketone; a nitrile such as acetonitrile or propionitrile; a carboxylic acid ester such as ethyl acetate or ethyl propionate; a nitrogen-containing non-protic polar solvent such as N,N-diemethylformamide, N,N-dimethylacetamide, N-methyl-2-pyrrolidone or 1,3-dimethyl-2-imidazolidinone; a sulfur-containing non-protic polar solvent such as dimethylsulfoxide or sulforane; a pyridine such as pyridine or picoline; or water may be mentioned. These solvents may be used alone, or two or more solvents may be used in combination. Preferred is a mixed solvent of a solvent selected from tetrahydrofuran and 1,4-dioxane, and water, and further preferred is a mixed solvent of tetrahydrofuran and water.

In a case where a mixed solvent of water and an organic solvent is used, the mixed proportion of the organic solvent to water is from 1:50 to 50:1, preferably from 1:10 to 10:1 by mass ratio.

The amount of (reaction concentration) of the reaction solvent to be used is not particularly restricted, and from 1 to 100 times by mass of the reaction solvent may be used per 1 part by mass of the aromatic nitro compound or the aromatic dinitro compound. The amount of the reaction solvent to be used is preferably from 1.5 to 50 times by mass, further preferably from 2.5 to 10 times by mass.

The reaction temperature is not particularly restricted, and for example, the reaction temperature is from -90 to 200°C, preferably from 0 to 150°C, further preferably from 10 to 120°C. The reaction time is usually from 0.05 to 200 hours, preferably from 0.5 to 100 hours.

The amount of stannous chloride to be used in the above reduction reaction is not particularly restricted, however, the amount of stannous chloride to be used is preferably from 1 to 10 times by mol, further preferably from 2 to 5 times by mol, per 1 mol of the nitro groups in the aromatic nitro compound represented by the formula 8. Further, in a case where there are two nitro groups such as the aromatic dinitro compound represented by the formula 1, the amount of stannous chloride is preferably from 2 to 20 times by mol, further preferably from 4 to 10 times by mol, per 1 mol of the aromatic dinitro compound.

Specifically, the reduction reaction is preferably carried out as described below. That is, an aromatic nitro compound, stannous chloride and a reaction solvent e.g. tetrahydrofuran as starting materials are charged into a reactor and stirred at preferably from 10 to 120°C for preferably from 1 to 20 hours.

As the charging order of the starting materials, the method of dividedly adding the aromatic compound or adding the reaction solvent having the aromatic nitro compound dissolved therein in a mixture of stannous chloride and the reaction solvent is preferred so as to avoid side reactions of reaction intermediates. The termination of the reaction can be confirmed by thin layer chromatography, high performance liquid chromatography or the like.

As described above, the aromatic nitro compound represented by the formula 8 or the aromatic dinitro compound represented by the formula 1 is reduced to form the aromatic amine compound represented by the formula 9 or the aromatic diamine compound represented by the formula 2, whereby a reaction liquid containing the aromatic amine, the unreacted starting material and the Sn compound can be obtained.

Next, a case where the compound B is an α-methylene-γ-butyrolactone compound represented by the formula 6 will be described (lactone ring-forming reaction).

As mentioned below, when a mixture of an acetal or ketal compound represented by the formula 4 as the compound A and a halogenated acrylic acid ester represented by the formula 5 is reacted in the presence of stannous chloride, an α-methylene-γ-butyrolactone compound represented by the formula 6 which is one type of the compound B is formed: 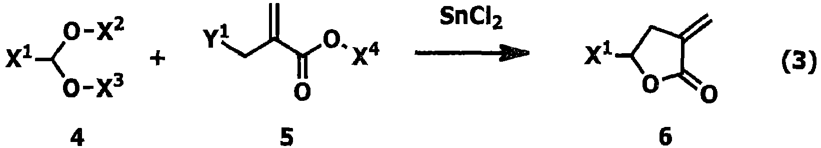 where X¹ is an organic group, X² and X³ are each independently a C₁₋₄ alkyl group, or X² and X³ may together form CH₂CH₂ or CH₂CH₂CH₂, X⁴ is a C₁₋₄ alkyl group, and Y¹ is Cl or Br.

The compound represented by the formula 4 to be used for forming the above lactone ring may, for example, be the compound represented by the formula 7: 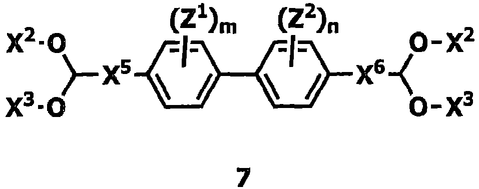 where X² and X³ are each independently a C₁₋₄ alkyl group, or X² and X³ may together form CH₂CH₂ or CH₂CH₂CH₂, X⁵ and X⁶ are each independently a C₁₋₁₀ alkylene group bonding to a benzene ring via an etheric bond or a single bond, Z¹ and Z² are each independently a substituent selected from a halogen atom, an alkyl group, a haloalkyl group, an alkoxy group and a haloalkoxy group, m and n are each independently an integer of from 0 to 4, and in a case where at least one of m and n is 2 or higher, two or more Z¹ and/or Z² may be the same or different from each other.

As the acrylic acid derivative represented by the formula 5 which is used in the above lactone ring-forming reaction, 2-(chloromethyl)acrylic acid, methyl 2-(chloromethyl)acrylate, ethyl 2-(chloromethyl)acrylate, 2-(bromomethyl)acrylic acid, methyl 2-(bromomethyl)acrylate, ethyl 2-(bromomethyl)acrylate or the like may be used.

The amount of the acrylic acid derivative represented by the formula 5 to be used is not particularly restricted, however, the amount of the acrylic acid derivative to be used is preferably from 1.0 to 1.5 times by mol, more preferably from 1.0 to 1.2 times by mol, per 1 mol of the acetal groups in the compound represented by the formula 4.

For example, in a case where the compound represented by the formula 4 has two acetal groups, the amount of the acrylic acid derivative represented by the formula 5 to be used is preferably from 2.0 to 3.0 times by mol, more preferably from 2.0 to 2.5 times by mol, per 1 mol of the acetal compound represented by the formula 4.

The tin compound used in the above lactone ring-forming reaction is generally stannous chloride, however, as another tin compound, a tin compound such as tin powder, tin chloride anhydride, tin chloride dihydrate or tin chloride pentahydrate may be used.

An acid may be added at the time of carrying out the reaction. As the acid, an inorganic acid such as hydrochloric acid, sulfuric acid, phosphoric acid or ammonium chloride or a salt thereof, an acidic resin such as Amberlyst 15, an organic acid such as p-toluene sulfonic acid, acetic acid or formic acid or a salt thereof may be used.

The above lactone ring-forming reaction may be carried out by using or not using a reaction solvent, however, the lactone ring-forming reaction is preferably carried out by using a reaction solvent. The reaction solvent is not particularly restricted, so long as it is inert to the reaction. For example, a hydrocarbon such as hexane, cyclohexane benzene or toluene, a halogenated hydrocarbon such as carbon tetrachloride, chloroform or 1,2-dichloroethane; an alcohol such as methanol, ethanol, or isopropyl alcohol; an ether such as diethyl ether, diisopropyl ether, 1,4-dioxane or tetrahydrofuran; a ketone such as an acetone, methyl ethyl ketone or methyl isobutyl ketone; a nitrile such as acetonitrile or propionitrile; a carboxylic acid ester such as ethyl acetate or ethyl propionate; a nitrogen-containing non-protic polar solvent such as N,N-dimethylformamide, N,N-dimethylacetamide, N-methyl-2-pyrrolidone or 1,3-dimethyl-2-imidazolidinone; a sulfur-containing non-protic polar solvent such as dimethyl sulfoxide or sulforane; a pyridine such as pyridine or picoline or water may be mentioned. Such a solvent may be used alone, or two or more of them may be mixed and used. Preferred is a mixed solvent of a solvent selected from tetrahydrofuran and 1,4-dioxane and water, and further preferred is a mixed solvent of tetrahydrofuran and water. In a case where a mixed solvent of water and an organic solvent is used, the mixed proportion of the organic solvent to water is from 1:50 to 50:1 by mass ratio, preferably from 1:10 to 10:1.

The amount (reaction concentration) of the reaction solvent to be used is not particularly restricted, and from 1 to 100 times by mass of the reaction solvent may be used, per 1 part by mass of the acetal or ketal represented by the formula 4. The amount of the reaction solvent to be used is preferably from 1.5 to 50 times by mass, further preferably from 2.5 to 50 times by mass.

The reaction temperature is not particularly restricted, however, the reaction temperature is usually from -90 to 200°C, preferably from 20 to 100°C. The reaction time is usually from 0.05 to 200 hours, preferably from 0.5 to 60 hours.

As described above, the α-methylene-γ-butyrolactone compound represented by the formula 6 is produced from a reaction mixture of the acetal or ketal compound represented by the formula 4 and the halogenated acrylic acid ester represented by the formula 5, whereby a reaction liquid containing the α-methylene-γ-butyrolactone compound, the unreacted starting materials and the Sn compound can be obtained.

The present invention is a method of bringing a reaction liquid obtained by reacting the compound A by means of stannous chloride into contact with a mixed solution of toluene and a potassium hydroxide aqueous solution to carry out liquid separation, efficiently removing the tin compound formed in the reaction liquid and obtaining the compound B from the organic layer at a high purity and a high yield.

Next, the obtained reaction liquid containing the compound B is subjected to a purification step of bringing the reaction liquid into contact with a mixed solution of a compound having a benzene ring and a potassium hydroxide aqueous solution to carry out liquid separation. The compound having a benzene ring preferably has a boiling point of from 80 to 170°C, more preferably from 80 to 150°C so as to be easily concentrated after the liquid separation. The compound having a benzene ring may, for example, be benzene, toluene, m-xylene, o-xylene, p-xylene, mesitylene, anisole, chlorobenzene or o-dichlorobenzene. Toluene is particularly preferred, since the toxicity is relatively low, and the distillation and drying can be easily carried out.

In the present invention, for example, in a case where the compound having a benzene ring is toluene, the following four methods may be mentioned as a purification step of separating the Sn compound from the reaction liquid containing the compound B to obtain the compound B as the desired product.

A method of adding a potassium hydroxide aqueous solution to a reaction liquid, followed by adding toluene to carry out liquid separation and obtaining the desired product from the organic layer (method 1), a method of adding toluene to a reaction liquid and washing with a potassium hydroxide aqueous solution to carry out liquid separated, and obtaining the desired product from the organic layer (method 2), a method of adding a reaction liquid into a container in which toluene and a potassium hydroxide aqueous solution are added to carry out liquid separation and obtaining the desired product from the organic layer (method 3) and a method of adding toluene and a potassium hydroxide aqueous solution in a reaction liquid at one time to carry out liquid separation and obtaining the desired product from the organic layer (method 4).

In the present invention, any method may be used, however, the above (method 3) is particularly preferred, since when mixing the potassium hydroxide aqueous solution, the temperature can be controlled at constant, the stirring failure due to the precipitation of the desired compound and the Sn compound during mixing will not be caused, and after mixing, the Sn compound will not be precipitated, and thereby the interface of two layers can be clearly distinguished.

The amount of potassium hydroxide in the potassium hydroxide aqueous solution used in the liquid separation is preferably an amount such that the Sn compound in the reaction liquid is made to be potassium hexahydrostannate having a high solubility, and the amount is preferably at least 3 times by mol, more preferably from 3 to 10 times by mol, particularly preferably from 3 to 6 times by mol, per 1 mol of tin atoms.

The concentration of the potassium hydroxide aqueous solution to be used is not particularly restricted, however, so long as the desired compound is not decomposed, the higher the concentration is, it is more preferred, since the treatment can be carried out with a small amount of the potassium hydroxide aqueous solution. If the desired product is decomposed, the treatment can be carried out by lowering the concentration of the potassium hydroxide aqueous solution. Thus, the concentration of the potassium hydroxide aqueous solution is preferably from 1 to 50 mass%, more preferably from 10 to 50 mass%.

The ratio in amount of the compound having a benzene ring to the potassium hydroxide aqueous solution is preferably from 100:1 to 1:100, more preferably from 75:1 to 1:75, particularly preferably from 50:1 to 1:50 by mass ratio, from the viewpoint of easily forming an interface at the time of the liquid separation and improving the volume efficiency.

The temperature in the liquid separation is not particularly restricted, so long as the desired product is not decomposed and is dissolved. The temperature is preferably from 10 to 80°C and from the viewpoint of easily controlling the temperature, more preferably from 10 to 60°C.

The time spent for the liquid separation is not particularly restricted, so long as the desired product is not decomposed, and an interface is formed, however, it is from 0.01 to 4 hours, preferably from 0.02 to 1 hour from the viewpoint of reducing working time.

The toluene layer obtained by the above liquid separation contains the desired product obtained by means of stannous chloride, and this organic layer may be concentrated as it is to separate and recover the desired product. Further, a poor solvent may be added to the organic layer to crystallize the desired product, and the desired product can be recovered by crystallization. In such a case, as the poor solvent, a hydrocarbon such as hexane, heptane or cyclohexane, an alcohol such as ethanol, methanol or i-propyl alcohol or the like is preferably used. Further, purification treatment such as recrystallization may be carried out in order to increase the purity of the desired product.

On the other hand, the water layer obtained by the liquid separation contains potassium hexahydrostannate having a high solubility and derived from stannous chloride used in the reduction reaction, etc., and the water layer may be discarded as it is, however, as the case requires, such a tin compound may be recovered.

### EXAMPLES

Now, the present invention will be described in further detail with reference to Examples. However, it should be understood that the present invention is by no means restricted thereto.

### EXAMPLE 1

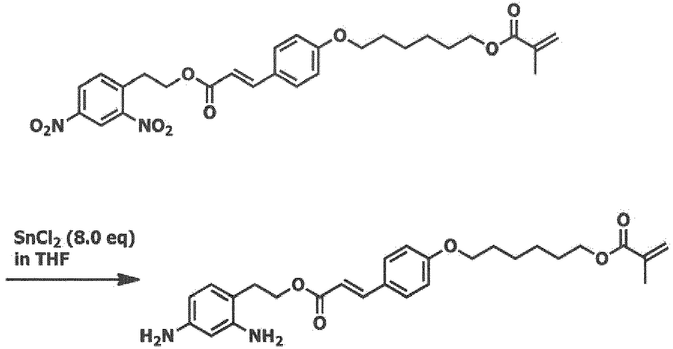

Stannous chloride (43.2 g, 228 mmol) was dissolved in a mixed solvent of THF (tetrahydrofuran) (45.0 g) and water (75.0 g), and a solution having (E)-4-(6-(methacryloyloxy)hexyloxy) cinnamic acid (2-(2,4-dinitrophenyl)ethyl)ester (15.0 g, 28.5 mmol) dissolved in THF (27.0 g) was dropwise added thereto at from 18 to 25°C over 1 hour. Then, the mixture was stirred for 25 hours to obtain a reaction mixture containing (E)-4-(6-(methacryloyloxy)hexyloxy) cinnamic acid (2-(2,4-diaminophenyl)ethyl)ester.

Then, the above reaction mixture was dropwise added to a mixed solution of toluene (75.0 g) and a 40 mass% potassium hydroxide aqueous solution (105 g). At that time, precipitates were not formed during the dropwise addition, and stirring could be successfully carried out. Then, the mixture was stirred for 10 minutes, the water layer was discarded, and a 6 mass% potassium hydroxide aqueous solution (75 g) was added to the obtained organic layer, followed by stirring. Then, the water layer was discarded. Then, a treatment of adding water (75 g), stirring for 10 minutes and discarding a water layer was repeated 5 times. Then, the organic layer was concentrated, and toluene was added so that the total amount would be 90.6 g, and precipitates were dissolved at 50°C. Then, the mixture was cooled to 0°C, and a precipitated solid was filtered and dried (40°C) to obtain the desired product of (E)-4-(6-methacryloyloxy)hexyloxy) cinnamic acid (2-(2,4-dinitrophenyl)ethyl)ester (pale brown solid, obtained amount: 11.0 g, yield: 83%, Sn content: 70 ppm).

Further, the above obtained compound (1.00 g) was dissolved in toluene (7.00 g) at 50°C, and 0.1 g of activated carbon (Special Shirasagi, manufactured by Japan EnviroChemicals, Ltd.) was added thereto, followed by stirring for 30 minutes. Then, the activated carbon was filtered out, and the filtrate was concentration. As a result, the Sn content in the desired product was lower than 1 ppm.

The obtained desired product was analyzed by HPLC by means of the following analyzing devices under the following analyzing conditions.
Device: LC-20A system (manufactured by Shimadzu Corporation)
Column: Inertsil ODS-3 (4.6 mm Φ × 250 mm, manufactured by GL Sciences Inc.)
Detector: UV detector (wavelength: 254 nm)
Eluent: acetonitrile/0.1 mass% acetic acid aqueous solution (70/30, v/v)

### COMPARATIVE EXAMPLE 1

5 g of ethyl acetate was added to a reaction mixture containing (E)-4-(6-(methacryloyloxy)hexyloxy) cinnamic acid (2-(2,4-diaminophenyl)ethyl)ester which was prepared in the same manner as in Ex. 1, except that the amount of (E)-4-(6-(methacryloyloxy)hexyloxy) cinnamic acid (2-(2,4-dinitrophenyl)ethyl)ester was changed to 1.0 g, and the proportions of the other starting materials were the same as those in Example 1. Then, sodium hydrogen carbonate (2.4 g) was added thereto. As a result, a large amount of white precipitates were formed, and thereby stirring could not be carried out.

### COMPARATIVE EXAMPLE 2

The reaction mixture containing (E)-4-(6-(methacryloyloxy)hexyloxy) cinnamic acid (2-(2,4-diaminophenyl)ethyl)ester was prepared in the same manner as in Comparative Example 1. 7.2 g, namely the amount of one third, of the reaction mixture was taken out, 3.3 g of ethyl acetate was added thereto, and further a 40 mass% sodium hydroxide aqueous solution was dropwise added thereto. When 1.3 g of the 40 mass% sodium hydroxide aqueous solution was added, a large amount of white precipitates were formed, and stirring could not be carried out. Further, 1.9 g of 40 mass% sodium hydroxide aqueous solution was dropwise added, and the precipitates were dissolved. However, a mixture was allowed to stand next three days, and precipitates were formed.

### COMPARATIVE EXAMPLE 3

A reaction mixture containing (E)-4-(6-(methacryloyloxy)hexyloxy) cinnamic acid (2-(2,4-diaminophenyl)ethyl)ester was prepared in the same manner as in Comparative Example 1. 5.0 g of ethyl acetate was added to the reaction mixture, and further a 40 mass% potassium hydroxide aqueous solution (7.0 g) was dropwise added. During the dropwise addition, white precipitates where formed, and the stirring property deteriorated. However, after the completion of dropwise adding the total amount, the precipitates were dissolved. Then, after allowing to stand for a few days, precipitates formed.

### EXAMPLE 2

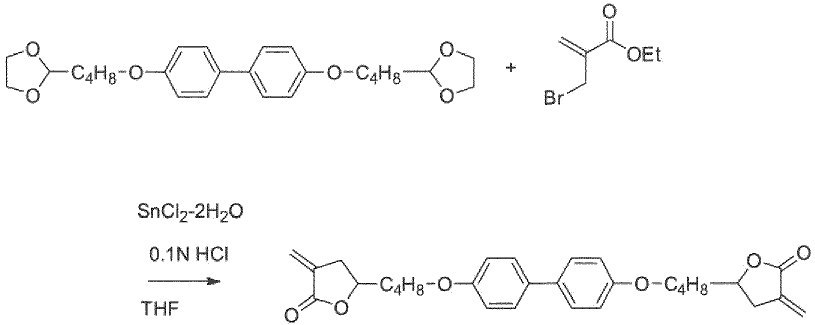

Stannous chloride dihydrate (78.52 g, 348 mmol) was dissolved in a mixed solution of THF (700.0 g), 4,4'-bis(4-(1,3-dioxolan-2-yl)butoxy)1,1'-biphenyl (70.0 g, 158 mmol) and bromo methacrylic acid ethyl ester (67.18 g, 348 mmol), and a 0.1 N hydrochloric acid aqueous solution (249.9 g) was dropwise added thereto at 64°C over 30 minutes. Then, the mixture was stirred for 24 hours to obtain a reaction mixture containing 4,4'-bis(4-(3-methylene tetrahydrofuran-2(3H)-on-5-yl)butoxy)biphenyl.

Then, a mixed solution of toluene (875.0 g) and THF (175.0 g) was added to the reaction mixture, followed by stirring at 50°C, and then, the hydrochloric acid layer was discarded. Then, the obtained organic layer was dropwise added to a 12.9 wt% potassium hydroxide aqueous solution (525.0 g) at 50°C over 30 minutes. Then, toluene (70.0 g) was further added thereto, followed by stirring at 50°C over 10 minutes, and then the water layer was discarded. A 5.2 wt% potassium hydroxide aqueous solution (525.0 g) was further added to the obtained organic layer, followed by stirring at 50°C for 10 minutes, and then the water layer was discarded. Then, a treatment of adding water (525.0 g), stirring at 50°C for 10 minutes and discarding the water layer was repeated 3 times to obtain an organic layer.

Further, activated carbon (7 g) (Special Shirasagi, manufactured by Japan EnviroChemicals, Ltd.) was added to the obtained organic layer, followed by stirring at 50°C for 30 minutes, and then the activated carbon was filtered out. Then, the filtrate was cooled to 5°C to precipitate crystals, followed by filtering. The obtained crystals were dried (40°C) to obtain 4,4'-bis(4-(3-methylene tetrahydrofuran-2(3H)-on-5-yl)butoxy)biphenyl (white solid, obtained amount: 64.59 g, yield: 83%). The Sn content was less than 1 ppm.

The obtained desired product was analyzed by HPLC by means of the following analyzing devices under the following analyzing conditions.
Device: LC-2010 system (manufactured by Shimadzu Corporation)
Column: Inertsil ODS-3 (4.6 mm Φ × 250 mm, manufactured by GL Sciences Inc.)
Detector: UV detector (wavelength: 265 nm)

Eluent acetonitrile/0.2 mass% ammonium acetate aqueous solution (70/30 (0 to 5 min.) → 85/15 (10 to 30 min.)) [v/v].

### EXAMPLE 3

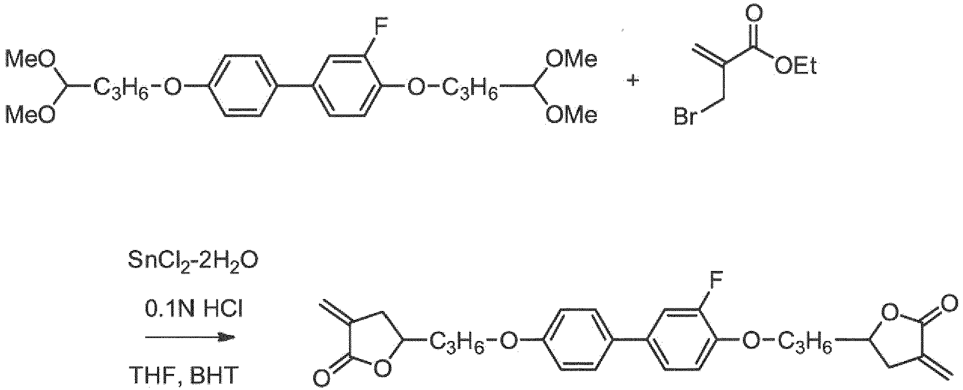

Stannous chloride dihydrate (124 g, 550 mmol) was dissolved in a mixed solution of THF (500.0 g), 4,4'-bis(4,4-dimethoxy butoxy)-3-fluoro-1,1'-biphenyl (100.0 g, 229 mmol), bromo methacrylic acid ethyl ester (97.29 g, 504 mmol) and dibutyl hydroxytoluene (0.56 g, 2.5 mmol), and a 0.1 N hydrochloric acid aqueous solution (175.0 g) was dropwise added thereto at 64°C over 30 minutes. Then, the mixture was stirred for 24 hours to obtain a reaction mixture containing 4,4'-bis(3-(3-methylenetetrahydrofuran-2(3H)-on-5-yl)propanoxy)-3-fluorobiphenyl.

Then, toluene (750.0 g) was added to the reaction mixture, followed by stirring at 50°C, and then the hydrochloric acid layer was discarded. Then, the obtained organic layer was dropwise added to a 12.9 mass% potassium hydroxide aqueous solution (750.0 g) at 50°C over 30 minutes. Then, the mixture was stirred at 50°C for 10 minutes, and the water layer was discarded. Further, a 5.2 wt% potassium hydroxide aqueous solution (750.0 g) was added to the organic layer, followed by stirring at 50°C for 10 minutes, and then the water layer was discarded. Then, a treatment of adding water (750.0 g), stirring at 50°C for 10 minutes and discarding the water layer was repeated 3 times to obtain an organic layer.

Further, activated carbon (10 g) (Special Shirasagi, manufactured by Japan EnviroChemicals, Ltd.) was added to the obtained organic layer, followed by stirring at 50°C for 30 minutes, and then the activated carbon was filtered out. Then, heptane (100.0 g) was dropwise added to the filtrate at 50°C over 30 minutes. Then, the filtrate was cooled to 5°C to precipitate crystals, followed by filtering. The obtained crystals were dried (40°C) to obtain 4,4'-bis(4-(3-methylene tetrahydrofuran-2(3H)-on-5-yl)propanoxy)-3-fluorobiphenyl (white solid, obtained amount: 87.36 g, yield: 79%). The Sn content was less than 1 ppm.

The obtained desired product was analyzed by HPLC in the same manner as in Example 2, except that acetonitrile/0.2 mass% ammonium acetate aqueous solution (70/30) [v/v] was used as the eluent.

### EXAMPLE 4

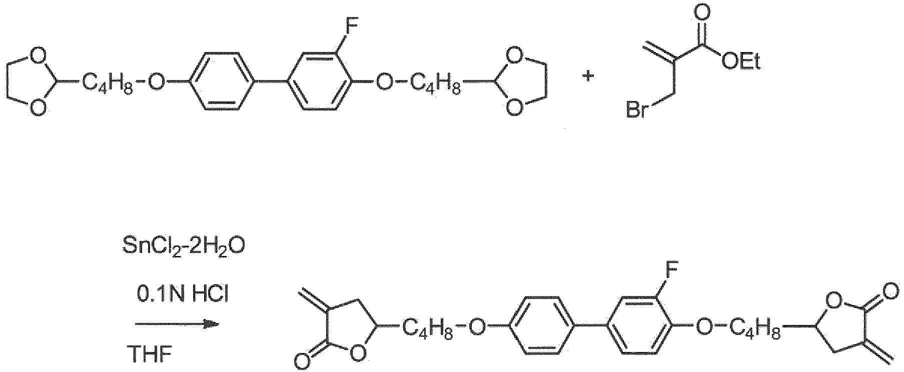

Stannous chloride dihydrate (129.35 g, 573 mmol) was dissolved in a mixed solution of THF (600.0 g), 4,4'-bis(4-(1,3-dioxolan-2-yl)butoxy)-3-fluoro-1,1'-biphenyl (120.0 g, 260 mmol) and bromo methacrylic acid ethyl ester (110.7 g, 573 mmol), and a 0.1 N hydrochloric acid aqueous solution (428.4 g) was dropwise added thereto at 64°C over 30 minutes. Then, the mixture was stirred for 24 hours to obtain a reaction mixture containing 4,4'-bis(4-(3-methylenetetrahydrofuran-2(3H)-on-5-yl)butoxy)-3-fluoro-biphenyl.

Further, the mixture was stirred at 50°C for 10 minutes, and then the hydrochloric acid layer was discarded. Then the obtained organic layer was dropwise added to a mixed solution of toluene (600.0 g) and a 12.9 mass% potassium hydroxide aqueous solution (900.0 g) at 50°C over 30 minutes. Then, the mixture was further stirred at 50°C for 10 minutes, and the water layer was discarded. Then, a 5.2 mass% potassium hydroxide aqueous solution (900.0 g) was added to the organic layer, followed by stirring at 50°C for 10 minutes, and then the water layer was discarded to obtain an organic layer.

Further, activated carbon (12 g) (Special Shirasagi, manufactured by Japan EnviroChemicals, Ltd.) was added to the obtained organic layer, followed by stirring at 50°C for 30 minutes, and then the activated carbon was filtered out. Then, heptane (360.0 g) was dropwise added to the filtrate at 50°C over 30 minutes. Then, the filtrate was cooled to 5°C to precipitate crystals, followed by filtering. The obtained crystals were dried (40°C) to obtain 4,4'-bis(4-(3-methylene tetrahydrofuran-2(3H)-on-5-yl)butoxy)-3-fluoro-biphenyl (white solid, obtained amount: 106.11 g, yield: 80%). The Sn content was less than 1 ppm.

The obtained desired product was analyzed by HPLC by means of the following analyzing devices under the following analysis conditions.
Device: LC-2010 system (manufactured by Shimadzu Corporation)
Column: Inertsil ODS-3 (4.6 mm Φ × 250 mm, manufactured by GL Sciences Inc.)
Detector: UV detector (wavelength: 265 nm)
Eluent: acetonitrile/0.2 mass% acetic acid aqueous solution (70/30, v/v) (0 to 5 min.) → 85/15 (10 to 30 min.)) [v/v] (the constant change in the composition was measured from 5 min to 10 min).

### INDUSTRIAL APPLICABILITY

According to the method of the present invention, in the reaction by means of SnCl₂ (stannous chloride), a Sn compound can be simply and efficiently removed, and the desired compound can be obtained at a high purity. Thus, the present invention is industrially useful.

## Claims

1. A method for producing a compound B, which comprises reacting a compound A as a starting material by means of stannous chloride in accordance with the following reaction formula (1) and which includes a purification step of bringing the above reaction liquid into contact with a mixed liquid of a compound having a benzene ring and a potassium hydroxide aqueous solution to carry out liquid separation and obtaining the compound B from the obtained organic layer: 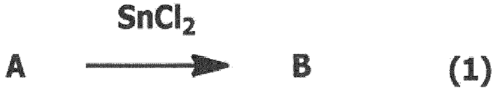 where the compound A may be a single compound or a mixture of plural compounds.

2. The method according to Claim 1, wherein the method of bringing the reaction liquid into contact with the mixed liquid of a compound having a benzene ring and a potassium hydroxide aqueous solution is a method of adding the reaction liquid into the mixed liquid of a compound having a benzene ring and a potassium hydroxide aqueous solution.

3. The method according to Claim 1 or 2, wherein the reaction represented by the reaction formula (1) is a reduction reaction or a lactone ring-forming reaction by means of stannous chloride.

4. The method according to Claim 3, wherein the compound A is an aromatic dinitro compound represented by the formula 1, and the compound B is an aromatic diamine compound represented by the formula 2: 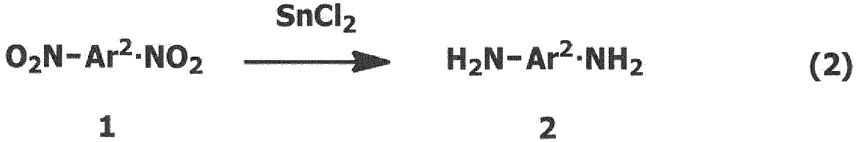 were Ar² is an aromatic group.

5. The method according to Claim 4, wherein the compound represented by the formula 1 is an aromatic dinitro compound represented by the formula 3:

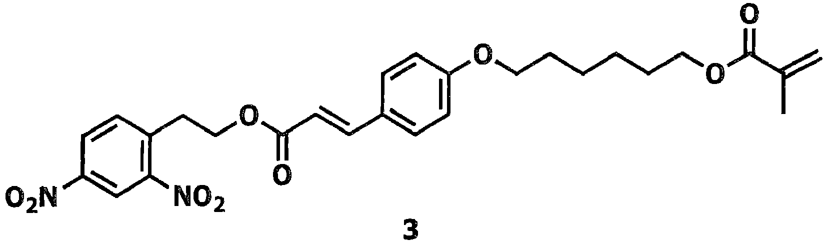

6. The method according to Claim 3, wherein the compound A is a mixture of an acetal compound represented by the formula 4 and an ester compound represented by the formula 5, and the compound B is an α-methylene-γ-butyrolactone compound: 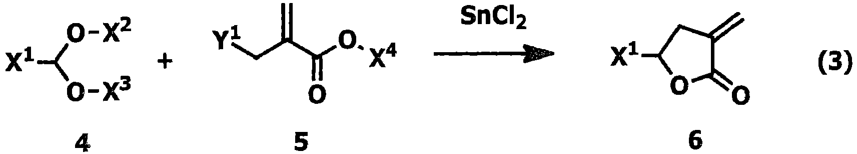 where X¹ is an organic group, X² and X³ are each independently a C₁₋₄ alkyl group, or X² and X³ may together form CH₂CH₂ or CH₂CH₂CH₂, X⁴ is a C₁₋₄ alkyl group, and Y¹ is Cl or Br.

7. The method according to Claim 6, wherein the compound represented by the formula 4 is an acetal compound represented by the formula 7: 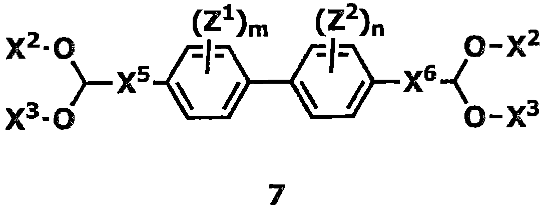 where X² and X³ are each independently a C₁₋₄ alkyl group, or X² and X³ may together form CH₂CH₂ or CH₂CH₂CH₂, X⁵ and X⁶ are each independently a C₁₋₁₀ alkylene group bonding to a benzene ring via an etheric bond or a single bond, Z¹ and Z² are each independently a substituent selected from a halogen atom, an alkyl group, a haloalkyl group, an alkoxy group and a haloalkoxy group, m and n are each independently an integer of from 0 to 4, and in a case where at least one of m and n is 2 or higher, two or more Z¹ and/or Z² may be the same or different from each other.

8. The method according to any one of Claims 1 to 7, wherein the concentration of the potassium hydroxide aqueous solution is from 1 to 50 mass%.

9. The method according to any one of Claims 1 to 8, wherein the amount of potassium hydroxide is at least 3 times by mol per 1 mol of tin atoms.

10. The method according to any one of Claims 1 to 9, wherein the temperature to carry out the liquid separation is from 10 to 80°C.

11. The method according to any one of Claims 1 to 10, wherein the ratio in amount to be used of the potassium hydroxide aqueous solution to the compound having a benzene ring is from 50:1 to 1:50 by mass ratio.

12. The method according to any one of Claims 1 to 11, wherein the compound having a benzene ring has a boiling point of from 80 to 170°C.

13. The method according to any one of Claims 1 to 12, wherein the compound having a benzene ring is toluene.

14. The method according to any one of Claims 1 to 13, wherein a reaction solvent is further used.

15. The method according to Claim 14, wherein the reaction solvent is tetrahydrofuran.

16. The method according to Claim 14, wherein the reaction solvent is water.

## Patentansprüche

1. Verfahren zur Herstellung einer Verbindung B, welches das Umsetzten einer Verbindung A als Ausgangsmaterial mittels Zinn(II)-chlorid in Übereinstimmung mit der folgenden Reaktionsformel (1) und welches einen Reinigungsschritt einschließt, in dem die obige Reaktionsflüssigkeit in Kontakt mit einer gemischten Flüssigkeit aus einer Verbindung, die einen Benzolring aufweist, und einer wässrigen Kaliumhydroxidlösung gebracht wird, um eine Flüssig-Trennung durchzuführen, und das Erhalten der Verbindung B aus der erhaltenen organischen Schicht umfasst: 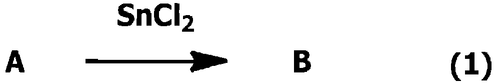 wobei die Verbindung A eine einzelne Verbindung sein kann oder eine Mischung aus mehreren Verbindungen.

2. Verfahren nach Anspruch 1, wobei das Verfahren des in Kontaktbringens der Reaktionsflüssigkeit mit der gemischten Flüssigkeit aus einer Verbindung, die einen Benzolring aufweist, und einer wässrigen Kaliumhydroxidlösung, ein Verfahren ist, in dem die Reaktionsflüssigkeit in die gemischte Flüssigkeit aus einer Verbindung, die einen Benzolring aufweist, und einer wässrigen Kaliumhydroxidlösung, gegeben wird.

3. Verfahren nach Anspruch 1 oder 2, wobei die Umsetzung, die durch die Reaktionsformel (1) wiedergegeben wird, eine Reduktionsreaktion ist oder eine Reaktion, bei der ein Lactonring mittels Zinn(II)-chlorid gebildet wird.

4. Verfahren nach Anspruch 3, wobei die Verbindung A eine aromatische Dinitroverbindung der Formel 1 ist und die Verbindung B eine aromatische Diaminverbindung der Formel 2 ist: 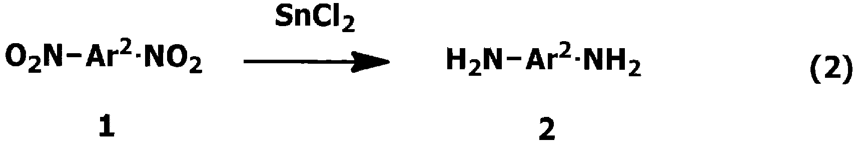 wobei Ar² für eine aromatische Gruppe steht.

5. Verfahren nach Anspruch 4, wobei die Verbindung der Formel 1 eine aromatische Dinitroverbindung der Formel 3 ist:

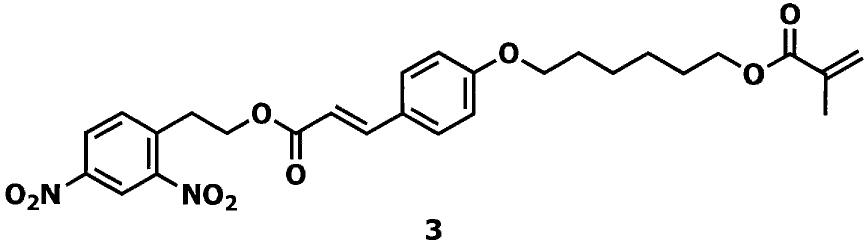

6. Verfahren nach Anspruch 3, wobei die Verbindung A eine Mischung aus einer Acetalverbindung der Formel 4 und einer Esterverbindung der Formel 5 ist und die Verbindung B eine α-Methylen-γ-butyrolacton-Verbindung ist: 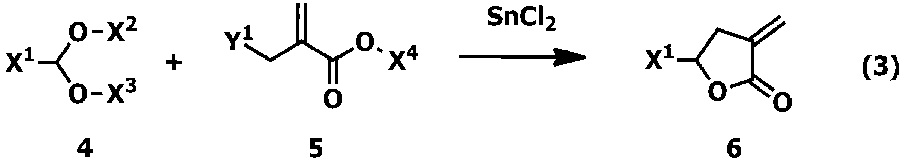 wobei X¹ für eine organische Gruppe steht, X² und X³ jeweils unabhängig für eine C₁₋₄ Alkylgruppe stehen oder X² und X³ zusammen CH₂CH₂ oder CH₂CH₂CH₂ bilden können, X⁴ für eine C₁₋₄ Alkylgruppe steht und Y¹ für Cl oder Br steht.

7. Verfahren nach Anspruch 6, wobei die Verbindung der Formel 4 eine Acetalverbindung der Formel 7 ist: 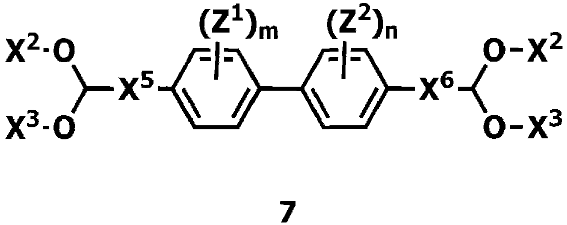 wobei X² und X³ jeweils unabhängig für eine C₁₋₄ Alkylgruppe stehen oder X² und X³ zusammen CH₂CH₂ oder CH₂CH₂ CH₂ bilden können, X⁵ und X⁶ jeweils unabhängig für eine C₁₋₁₀ Alkylengruppe stehen, die an einen Benzolring über eine Etherbindung oder eine Einzelbindung bindet, Z¹ und Z² jeweils unabhängig für einen Substituent stehen, ausgewählt aus einem Halogenatom, einer Alkylgruppe, einer Haloalkylgruppe, einer Alkoxygruppe und einer Haloalkoxygruppe, m und n jeweils unabhängig für eine ganze Zahl von 0 bis 4 stehen, und in dem Fall, in dem wenigstens eines von m und n 2 oder mehr ist, können zwei oder mehr Z¹ und/oder Z² gleich oder voneinander verschieden sein.

8. Verfahren nach einem der Ansprüche 1 bis 7, wobei die Konzentration der wässrigen Kaliumhydroxidlösung von 1 bis 50 Masseprozent beträgt.

9. Verfahren nach einem der Ansprüche 1 bis 8, wobei die Menge des Kaliumhydroxids wenigstens 3-fach nach Mol pro 1 Mol Zinnatome beträgt.

10. Verfahren nach einem der Ansprüche 1 bis 9, wobei die Temperatur, um die Flüssig-Trennung durchzuführen, von 10 bis 80°C beträgt.

11. Verfahren nach einem der Ansprüche 1 bis 10, wobei das Verhältnis der zu verwendenden Menge der wässrigen Kaliumhydroxidlösung zu der Verbindung, die den Benzolring aufweist, von 50:1 bis 1:50 nach Massenverhältnis beträgt.

12. Verfahren nach einem der Ansprüche 1 bis 11, wobei die Verbindung, die den Benzolring aufweist, einen Siedepunkt von 80 bis 170°C aufweist.

13. Verfahren nach einem der Ansprüche 1 bis 12, wobei die Verbindung, die den Benzolring aufweist, Toluol ist.

14. Verfahren nach einem der Ansprüche 1 bis 13, wobei ferner ein Reaktionslösungsmittel verwendet wird.

15. Verfahren nach Anspruch 14, wobei das Reaktionslösungsmittel Tetrahydrofuran ist.

16. Verfahren nach Anspruch 14, wobei das Reaktionslösungsmittel Wasser ist.

## Revendications

1. Procédé pour produire un composé B, qui comprend la réaction d'un composé A servant de matériau de départ au moyen de chlorure stanneux conformément à la formule réactionnelle (1) qui suit et qui comprend une étape de purification de mise en contact du liquide réactionnel ci-dessus avec un mélange liquide d'un composé ayant un cycle benzène et d'une solution aqueuse d'hydroxyde de potassium pour que soit mise en œuvre une séparation de liquides, et d'obtention du composé B à partir de la phase organique obtenue : 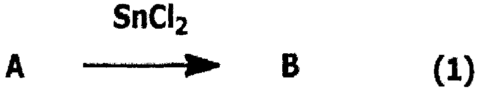 où le composé A peut être un seul composé ou un mélange de plusieurs composés.

2. Procédé selon la revendication 1, dans lequel le procédé de mise en contact du liquide réactionnel en contact avec le mélange liquide d'un composé ayant un cycle benzène et d'une solution aqueuse d'hydroxyde de potassium est un procédé d'addition du liquide réactionnel dans le mélange liquide d'un composé ayant un cycle benzène et d'une solution aqueuse d'hydroxyde de potassium.

3. Procédé selon la revendication 1 ou 2, dans lequel la réaction représentée par la formule réactionnelle (1) est une réaction de réduction ou une réaction de formation de cycle lactone au moyen de chlorure stanneux.

4. Procédé selon la revendication 3, dans lequel le composé A est un composé dinitro aromatique représenté par la formule 1, et le composé B est un composé diamine aromatique représenté par la formule 2 : 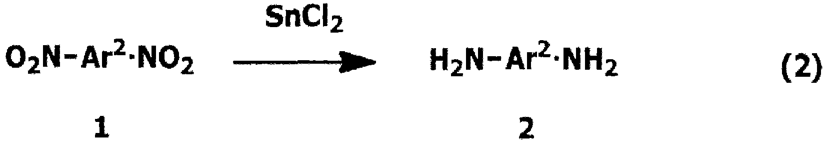 où Ar² est un groupe aromatique.

5. Procédé selon la revendication 4, dans lequel le composé représenté par la formule 1 est un composé dinitro aromatique représenté par la formule 3 :

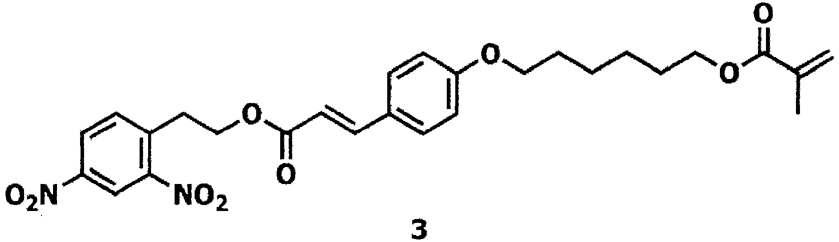

6. Procédé selon la revendication 3, dans lequel le composé A est un mélange d'un composé acétal représenté par la formule 4 et d'un composé ester représenté par la formule 5, et le composé B est un composé α-méthylène-γ-butyrolactone : 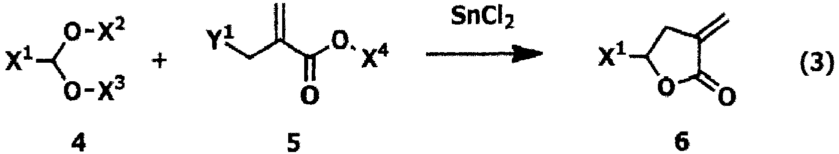 où X¹ est un groupe organique, X² et X³ sont chacun indépendamment un groupe alkyle en C₁ à C₄, ou X² et X³ peuvent former ensemble CH₂CH₂ ou CH₂CH₂CH₂, X⁴ est un groupe alkyle en C₁ à C₄, et Y¹ est Cl ou Br.

7. Procédé selon la revendication 6, dans lequel le composé représenté par la formule 4 est un composé acétal représenté par la formule 7 : 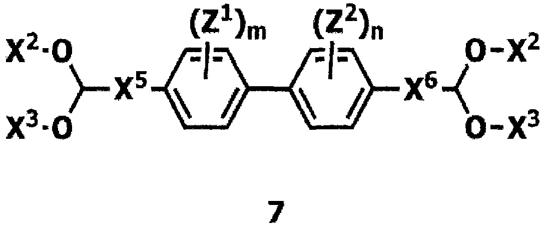 où X² et X³ sont chacun indépendamment un groupe alkyle en C₁ à C₄, ou X² et X³ peuvent former ensemble CH₂CH₂ ou CH₂CH₂CH₂, X⁵ et X⁶ sont chacun indépendamment un groupe alkylène en C₁ à C₁₀ se liant à un cycle benzène via une liaison éther ou une liaison simple, Z¹ et Z² sont chacun indépendamment un substituant choisi parmi un atome d'halogène, un groupe alkyle, un groupe halogénoalkyle, un groupe alcoxy et un groupe halogénoalcoxy, m et n sont chacun indépendamment un entier de 0 à 4, et dans le cas où au moins l'un parmi n et n vaut 2 ou plus, deux ou plus de deux Z¹ et/ou Z² peuvent être identiques ou différents.

8. Procédé selon l'une quelconque des revendications 1 à 7, dans lequel la concentration de la solution aqueuse d'hydroxyde de potassium est de 1 à 50 % en masse.

9. Procédé selon l'une quelconque des revendications 1 à 8, dans lequel la quantité d'hydroxyde de potassium est d'au moins 3 moles par mole d'atomes d'étain.

10. Procédé selon l'une quelconque des revendications 1 à 9, dans lequel la température pour la mise en œuvre de la séparation de liquides est de 10 à 80°C.

11. Procédé selon l'une quelconque des revendications 1 à 10, dans lequel le rapport en quantité à utiliser de la solution aqueuse d'hydroxyde de potassium sur le composé ayant un cycle benzène est de 50/1 à 1/50 en rapport en masse.

12. Procédé selon l'une quelconque des revendications 1 à 11, dans lequel le composé ayant un cycle benzène a un point d'ébullition de 80 à 170°C.

13. Procédé selon l'une quelconque des revendications 1 à 12, dans lequel le composé ayant un cycle benzène est le toluène.

14. Procédé selon l'une quelconque des revendications 1 à 13, dans lequel un solvant réactionnel est en outre utilisé.

15. Procédé selon la revendication 14, dans lequel le solvant réactionnel est le tétrahydrofurane.

16. Procédé selon la revendication 14, dans lequel le solvant réactionnel est l'eau.
